(19) Europäisches Patentamt
European Patent Office
Office européen des brevets

(11) **EP 4 477 147 A1**

(12) **EUROPEAN PATENT APPLICATION**

(43) Date of publication:
**18.12.2024 Bulletin 2024/51**

(21) Application number: **24181827.7**

(22) Date of filing: **12.06.2024**

(51) International Patent Classification (IPC):
**A61B 5/145** (2006.01)   **A61B 5/1495** (2006.01)
**A61B 5/00** (2006.01)   **A61B 5/1486** (2006.01)

(52) Cooperative Patent Classification (CPC):
**A61B 5/14532; A61B 5/14865; A61B 5/1495;**
**A61B 5/7221;** A61B 2560/0223; A61B 2560/045

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **13.06.2023 KR 20230075334**

(71) Applicant: **I-SENS, INC.**
**Seoul 06646 (KR)**

(72) Inventor: **LEE, Da-vid**
**06646 Seoul (KR)**

(74) Representative: **HGF**
**HGF Limited**
**1 City Walk**
**Leeds LS11 9DX (GB)**

(54) **METHOD FOR CALIBRATING SENSITIVITY FOR GLUCOSE MEASUREMENT**

(57)   One embodiment may provide a method for calibrating sensitivity for glucose measurement, the method comprising: obtaining an offset value for determining sensitivity; determining a first sensitivity from a sensor data and a reference glucose value, based on the offset value; determining a second sensitivity from the first sensitivity and a predetermined sensitivity depending on a sensor; determining a third sensitivity by adjusting the second sensitivity based on reliability of the first sensitivity that is based on a difference between the first sensitivity and a previous sensitivity; determining a fourth sensitivity from the third sensitivity based on whether a glucose value obtained from the offset value and the third sensitivity is within a specific range; and determining the fourth sensitivity as the sensitivity.

FIG 1

EP 4 477 147 A1

# Description

## CROSS-REFERENCE TO RELATED APPLICATION

This application claims priority to and the benefit of Korean Patent Application No. 10-2023-0075334, filed on June 13, 2023, the disclosure of which is incorporated herein by reference in its entirety.

## BACKGROUND

### 1. Field of the Invention

The present embodiments relate to calibrating the sensitivity for continuous blood glucose (hereinafter, simply referred to as glucose) measurement, and more particularly, to a technology that includes multiple steps for adjusting sensitivity to calibrate the sensitivity.

### 2. Discussion of Related Art

With recent advances in medical technology, various medical devices that are attached to the user's body have been developed and commercialized. Medical devices attached to the user's body may be attached to the skin of patients with chronic illnesses and usefully utilized in monitoring biometric information or performing treatment.

For example, chronic illnesses such as diabetes require constant care, and a medical device attached to the skin to measure glucose may be used in monitoring glucose in a patient with diabetes. Diabetes is characterized by almost no noticeable symptoms in the early stages, but as the disease progresses, diabetes-specific symptoms such as polydipsia, polyphagia, polyuria, weight loss, general malaise, itchy skin, and slow-healing wounds on the hands and feet appear. As diabetes progresses further, complications that lead to vision impairment, hypertension, kidney disease, stroke, periodontal disease, muscle cramps, neuralgia, and gangrene appear. In order to diagnose diabetes and manage diabetes to prevent diabetes from leading to complications, systematic glucose measurement and treatment should be carried out together.

For diabetic patients and people who have not developed diabetes but have a higher than normal glucose level detected in their blood, numerous medical device manufacturers provide various types of glucose meters that can measure glucose.

There are two types of glucose meters: one that collects blood from the user's fingertip to measure glucose on a one-time basis, and the other that is attached to the user's stomach or arm to continuously measure glucose.

Diabetic patients generally go back and forth between hyperglycemia and hypoglycemia. Emergency situations occur during hypoglycemia, and a patient may lose consciousness, or prolonged hypoglycemia without sugar supply may lead to death. Therefore, immediate detection of hypoglycemia is very important for diabetic patients, but invasive glucose meters that measure glucose intermittently have limitations in accurately detecting hypoglycemia.

Recently, to overcome such limitations, a continuous glucose monitoring system (CGMS), which is inserted into the human body to measure glucose levels at intervals of several minutes, has been developed and used. In order to minimize the user's pain and resistance following blood collection, the CGMS may measure glucose continuously after a needle-type transcutaneous sensor is inserted into areas where pain is relatively less, such as the stomach and arms.

The CGMS includes a sensor transmitter inserted into the user's skin and configured to measure glucose in the body and transmit the measured glucose level and a terminal configured to output the received glucose level.

Here, since glucose is measured while the sensor transmitter is attached to the body and a part of a sensor of the sensor transmitter is inserted into the body, the sensor inserted into the body may be perceived as foreign matter. That is, a biological response to the sensor- for example, a hydration reaction- may occur. As a result, sensitivity (slope) may change at the beginning when the sensor is inserted. Consequently, the accuracy of the sensor may decrease, and it may be difficult to maintain the usage period of the sensor. The changed slope may cause an excessively large or excessively small glucose value to be obtained(or calculated) for a measured biometric signal and thus may decrease the accuracy of the CGMS. Conventionally, in order to compensate for such a change in sensitivity (slope), a method in which a predetermined offset value and simple sensitivity divided into a reference glucose value input by the user- a glucose value measured by a blood glucose monitoring system (BGMS)- and a current value of a sensor are used and a method in which sensitivity is determined through regression analysis for a reference glucose value and a current value corresponding thereto have been developed. However, such methods have a problem in that inaccurate sensitivity and offset value may be provided due to a failure to sensitively respond to an abnormal glucose value or an abnormal operation of the sensor.

## SUMMARY OF THE INVENTION

Against this background, the present embodiments are directed to providing a technology for calibrating sensitivity to obtain accurate sensitivity.

Against this background, the present embodiments are also directed to providing a technology for providing calibrated sensitivity by adjusting sensitivity through several steps to increase the accuracy of sensitivity.

To achieve the above objectives, one embodiment may provide a method for calibrating sensitivity for glucose measurement, the method including: obtaining an offset value for determining sensitivity; based on the offset value, determining a first sensitivity from a sensor data and a reference glucose value; determining a second sensitivity from the first sensitivity and predetermined sensitivity depending on a sensor; determining a third sensitivity by adjusting the second sensitivity based on reliability of the first sensitivity that is based on a difference between the first sensitivity and a previous sensitivity; determining a fourth sensitivity from the third sensitivity based on whether a glucose value obtained from the offset value and the third sensitivity is within a specific range; and determining the fourth sensitivity as the sensitivity.

Another embodiment may provide a method for calibrating sensitivity for glucose measurement, the method including: obtaining an offset value; based on the offset value, determining a sensitivity from a sensor data and a reference glucose value; and adjusting the sensitivity according to at least one of a first method in which the sensitivity is adjusted based on a predetermined sensitivity depending on a sensor, a second method in which the sensitivity is adjusted based on a difference between the sensitivity and a previous sensitivity, and a third method in which the sensitivity is adjusted based on whether a glucose value obtained from the sensor data, in which the offset value is reflected, and the sensitivity is within a specific range.

BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:

FIG. 1 is a view for schematically describing a glucose monitoring system according to one embodiment;
FIG. 2 is a view for describing an applicator for attaching a sensor transmitter to the human body according to one embodiment;
FIG. 3 is a view for describing a process of attaching the sensor transmitter to the human body using the applicator according to one embodiment;
FIG. 4 is a block diagram of the sensor transmitter according to one embodiment;
FIG. 5 is a block diagram of a terminal according to one embodiment;
FIG. 6 is a flowchart illustrating a method for calibrating sensitivity according to one embodiment;
FIG. 7 is an exemplary view of adjusting sensitivity based on coding information of a sensor according to one embodiment;
FIG. 8 is an exemplary view of adjusting sensitivity based on a difference from a previous sensitivity according to one embodiment;
FIGS. 9 to 11 are exemplary views of adjusting sensitivity based on a specific range formed by a reference glucose value and a previous glucose value obtained from a previous sensitivity according to one embodiment; and
FIG. 12 is a flowchart illustrating a method for calibrating sensitivity according to another embodiment.

DETAILED DESCRIPTION OF EXEMPLARY EMBODIMENTS

In describing the present invention, when it is determined that relevant known functions that are apparent to those of ordinary skill in the art may unnecessarily obscure the gist of the present invention, detailed description thereof will be omitted.

Terms used in the present application are only used to describe specific embodiments and are not intended to limit the present invention. A singular expression includes a plural expression unless the context clearly indicates otherwise. In the present application, terms such as "include" or "have" are intended to designate the presence of features, numbers, steps, operations, components, parts, or combinations thereof mentioned herein and should not be understood as precluding the possibility of presence or addition of one or more other features, numbers, steps, operations, components, parts, or combinations thereof.

Hereinafter, embodiments according to the present invention will be described in detail with reference to the accompanying drawings, and in describing the embodiments with reference to the accompanying drawings, the same or corresponding components will be denoted by the same reference numerals, and redundant description thereof will be omitted.

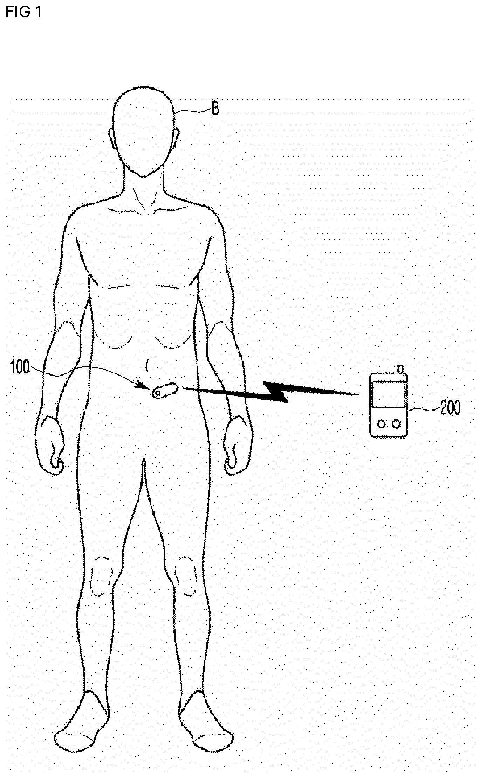

FIG. 1 is a view for schematically describing a glucose monitoring system according to one embodiment.

Referring to FIG. 1, a CGMS 10 (hereinafter referred to as "system") according to one embodiment may include a sensor transmitter 100 and a terminal 200.

The sensor transmitter 100 is attached to a human body B, and when the sensor transmitter 100 is attached to the human body B, one end of a sensor of the sensor transmitter 100 may be inserted into the skin and periodically extract body fluids from the human body to measure glucose.

The terminal 200 may receive a biometric signal including glucose information from the sensor transmitter 100, generate glucose information from the biometric signal, and output the glucose information to a user. Examples of the terminal 200 may include various devices such as a smartphone, a mobile phone, a tablet personal computer (PC), a desktop, and a laptop, but the terminal 200 is not limited thereto and may be any other device which has a communication interface that can communicate with the sensor transmitter 100 and in which a program or an application may be installed.

The sensor transmitter 100 may transmit a periodically measured biometric signal to the terminal 200 in response to a request of the terminal 200 or at each set time. For data communication between the sensor transmitter 100 and the terminal 200, the sensor transmitter 100 and the terminal 200 may be connected to each other via a wire by a universal serial bus (USB) cable or the like or may be wirelessly connected to each other by methods such as infrared communication, near-field communication (NFC), or Bluetooth.

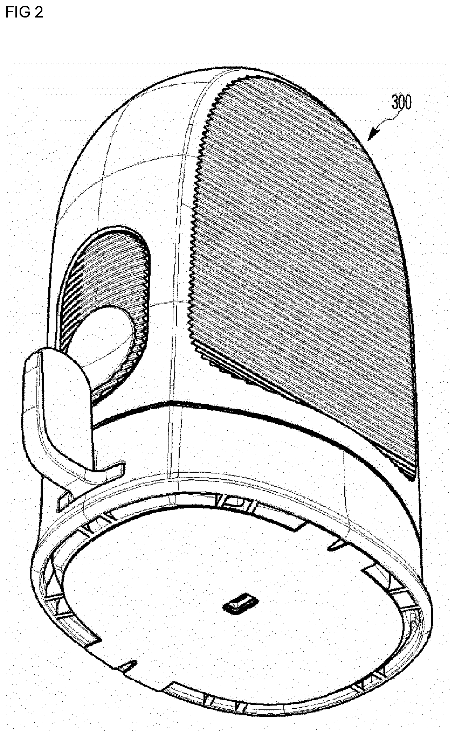

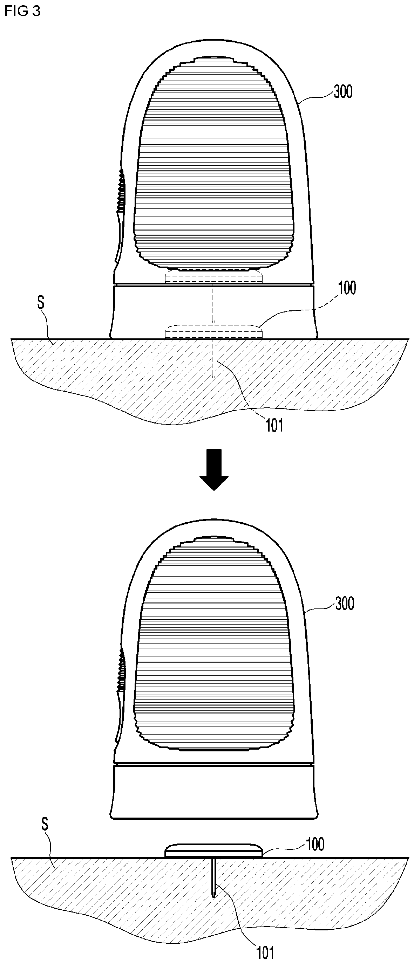

FIG. 2 is a view for describing an applicator for attaching a sensor transmitter to the human body according to one embodiment, and FIG. 3 is a view for describing a process of attaching the sensor transmitter to the human body using the applicator according to one embodiment.

Referring to FIGS. 2 and 3, an applicator 300 according to one embodiment has the sensor transmitter 100 disposed therein and operates to attach the sensor transmitter 100, which is discharged to the outside due to manipulation by the user, to a specific body part of the user. The applicator 300 is formed in a shape that has one open surface, and the sensor transmitter 100 is installed in the applicator 300 through the one open surface of the applicator 300.

In order to insert one end of the sensor provided in the sensor transmitter 100 into the skin when the sensor transmitter 100 is attached to a part of the body using the applicator 300, the applicator 300 may include a needle (not illustrated) formed to have the one end of the sensor surrounded therein, a first elastic member (not illustrated) configured to push both the needle and the one end of the sensor toward the skin, and a second elastic member (not illustrated) configured to withdraw only the needle. With such a configuration of the applicator 300, by decompressing the first elastic member (not illustrated) that is disposed in a compressed state in the applicator 300, the needle and the one end of the sensor may be simultaneously inserted into the skin. When the one end of the sensor is inserted into the skin, only the needle is withdrawn by decompressing the second elastic member (not illustrated) that is in a compressed state. The user may safely and easily attach the sensor transmitter 100 to the skin using the applicator 300.

A process of attaching the applicator 300 to the human body B will be described in detail. In a state in which a protective cap (not illustrated) is removed, the one open surface of the applicator 300 is brought into close contact with the skin S of a specific part of the human body B. When the applicator 300 is operated in the state in which the applicator 300 is brought into close contact with the skin S of the human body B, the sensor transmitter 100 may be discharged from the applicator 300 and attached to the skin S. Here, one end of a sensor 101 is disposed to be exposed from the sensor transmitter 100 at a lower portion of the sensor transmitter 100, and the one end of the sensor 101 may be partially inserted into the skin S through the needle disposed in the applicator 300. Accordingly, the sensor transmitter 100 may be attached to the skin S while the one end of the sensor 101 is inserted into the skin S.

Here, for the sensor transmitter 100 to be fixed and attached to the skin S of the human body B, an adhesive tape may be disposed on a surface of the sensor transmitter 100 that comes in contact with the human body B. Accordingly, when the applicator 300 is moved away from the skin S of the human body B, the sensor transmitter 100 may remain fixed and attached to the skin S of the human body B due to the adhesive tape.

Then, when power is applied to the sensor transmitter 100, the sensor transmitter 100 may communicate with a terminal and transmit a biometric signal including glucose information to the terminal. The sensor transmitter 100 may generate various pieces of biometric information other than glucose information, but hereinafter, a case in which glucose information, which is an example of biometric information, is measured will be described.

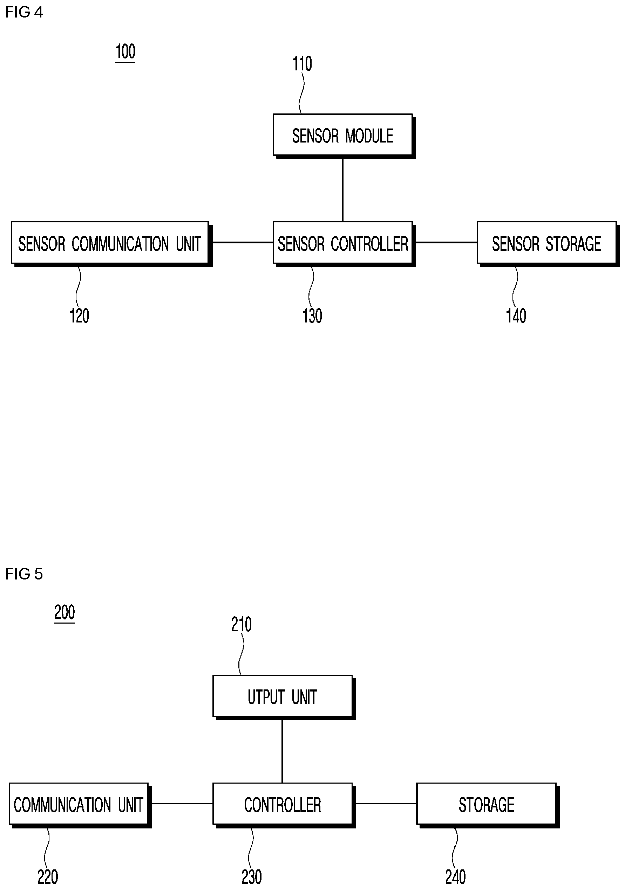

FIG. 4 is a block diagram of the sensor transmitter according to one embodiment.

Referring to FIG. 4, the sensor transmitter 100 according to one embodiment may include a sensor module 110, a sensor communication unit 120, a sensor controller 130, and a sensor storage 140.

The sensor module 110 may include at least one sensor inserted into the human body to detect biomass. The at least one sensor may measure biomass and generate a biometric signal. The biometric signal may be an analog signal that includes a current value.

The sensor communication unit 120 may exchange data or information with a terminal. For example, the sensor communication unit 120 may transmit a biometric signal received from the sensor module 110 or data stored in the sensor storage 140 to the terminal.

The sensor controller 130 may control the overall configuration of the sensor transmitter 100 including the sensor module 110, the sensor storage 140, and the sensor communication unit 120. For example, the sensor controller 130 may receive a control signal from the terminal and control the configuration of the sensor transmitter 100 according to the control signal. Also, the sensor controller 130 may process a biometric signal. For example, the sensor controller 130 may convert a biometric signal into an analog or digital form or may perform processing for removing noise as necessary.

Date or information may be stored in the sensor storage 140. For example, data relating to biomass measured by the sensor module 110- for example, a current value of a biometric signal- or data received from the terminal- for example, a command value of a control signal- may be stored in the sensor storage 140.

FIG. 5 is a block diagram of a terminal according to one embodiment.

Referring to FIG. 5, the terminal 200 according to one embodiment may include an output unit 210, a communication unit 220, a controller 230, and a storage 240.

The output unit 210 may output biometric information included in a biometric signal- for example, glucose information- for the user to check the biometric information. For example, the output unit 210 may display glucose information in numerical values or a graph processed from the numerical values.

The communication unit 220 may communicate with the sensor communication unit of the sensor transmitter and exchange data or information therewith. For example, the communication unit 220 may receive a biometric signal including information on biomass measured by the sensor transmitter- biometric information. Here, the communication unit 220 may receive a primarily processed biometric signal from the sensor transmitter. Alternatively, the communication unit 220 may transmit a control signal for controlling the sensor transmitter to the sensor transmitter.

Data or information may be stored in the storage 240. For example, data received from the sensor transmitter- for example, biometric information- may be stored in the storage 240. Here, the biometric information may include glucose information and may have the form of a current value. Alternatively, data input from the user or environment setting data for setting an operating environment of the terminal may be stored in the storage 240.

The controller 230 may include at least one processor configured to execute a program that calibrates sensitivity for glucose measurement and at least one memory in which the program is stored. The memory and the processor included in the controller 230 may be integrated on a single chip or physically separated.

The memory may be implemented as a non-volatile memory device such as a read only memory (ROM), a programmable ROM (PROM), an erasable programmable ROM (EPROM), an electrically erasable programmable ROM (EEPROM), and a flash memory or a volatile memory device such as a random access memory (RAM) to store various programs, data, and/or information.

Meanwhile, the controller 230 may generate a glucose value, which is glucose information formed into a numerical value, from biometric information. To this end, the controller 230 may obtain biometric information in the form of a current value from the sensor transmitter and may perform preprocessing and/or processing of the current value of the biometric information. The controller 230 may first obtain sensitivity and generate a glucose value based on the sensitivity.

The controller 230 may determine sensitivity based on the current value and a reference glucose value that is input from the outside, and the sensitivity may be determined according to Mathematical Equation 1 below. Here, C represents a current value at a point in time at which sensitivity is determined based on a reference glucose value, that is, a point in time at which sensitivity is calibrated, and BGr represents a reference glucose value, which is a glucose value that is obtained from a blood glucose monitoring system (BGMS) and does not necessarily need to be obtained at the point in time at which sensitivity is calibrated.

[Mathematical Equation 1]

$$S = \frac{BGr}{C}$$

After sensitivity is obtained as above, the controller 230 may multiply the sensitivity by a current value and generate a glucose value that corresponds to the corresponding current value. The controller 230 may provide this new glucose value to the user through the output unit 210. For example, the controller 230 may obtain sensitivity as in Mathematical Equation 1 through a first current value obtained at a first point in time and one reference glucose value, and then obtain a new glucose value using this sensitivity until a point in time at which a subsequent sensitivity calibration occurs- a second point in time. Then, the controller 230 may calibrate sensitivity again at the second point in time. The controller 230 may obtain sensitivity as in Mathematical Equation 1 through a second current value obtained at the second point in time and another reference glucose value and may newly obtain a glucose value corresponding thereto.

Meanwhile, the sensitivity may vary according to various environment variables. For example, a negative intercept phenomenon in which a variation in current values or a decrease in current values, which occurs due to hydration occurring when the sensor is inserted into the living body, continues may be a factor that changes sensitivity. When the sensor transmitter transmits a current value decreased from the original to the terminal 200 due to the negative intercept phenomenon, the controller 230 of the terminal 200 may obtain a sensitivity from the current value lower than the original according to Mathematical Equation 1. The sensitivity obtained in this case may greatly increase. When the controller 230 obtains a glucose value with excessively high sensitivity, the glucose value may increase even at a minute current value, and the controller 230 may generate an inaccurate glucose value.

In order to correct such an error, the controller 230 may calculate an offset value and reflect the offset value in obtaining sensitivity. The controller 230 may obtain the offset value in various ways without limitation, and the offset value may be fixed or variable. According to Mathematical Equation 2 below, the controller 230 may determine a sensitivity in which the offset value is reflected. Here, Voff represents an offset value that is predetermined by the user or changed by an algorithm of the storage 240.

[Mathematical Equation 2]

$$S = \frac{BGr}{C - Voff}$$

In the example described above, the decrease in the current value due to the negative intercept phenomenon may be offset by the offset value, a decreased denominator may increase, and sensitivity may also change to a non-excessive, appropriate level. However, the controller 230 of the terminal 200 according to one embodiment may, instead of immediately utilizing the sensitivity in which the offset value is reflected in obtaining a glucose value, adjust the sensitivity, and then finally utilize the adjusted sensitivity in obtaining a glucose value. Hereinafter, a method in which the controller 230 of the terminal 200 calibrates sensitivity through multiple steps for adjusting sensitivity will be described.

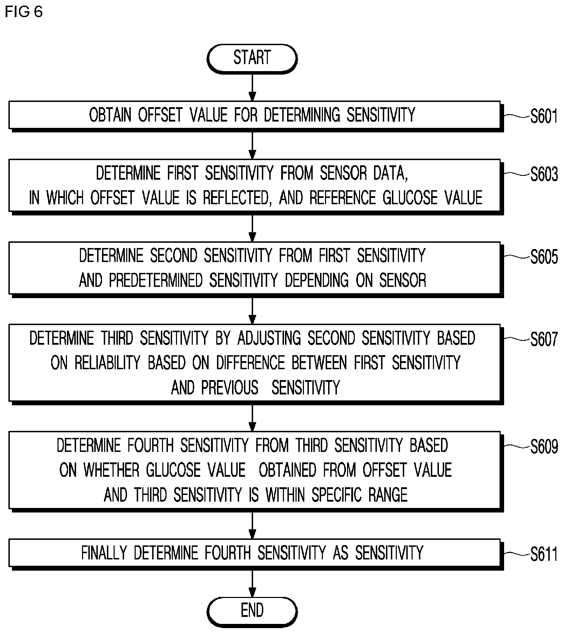

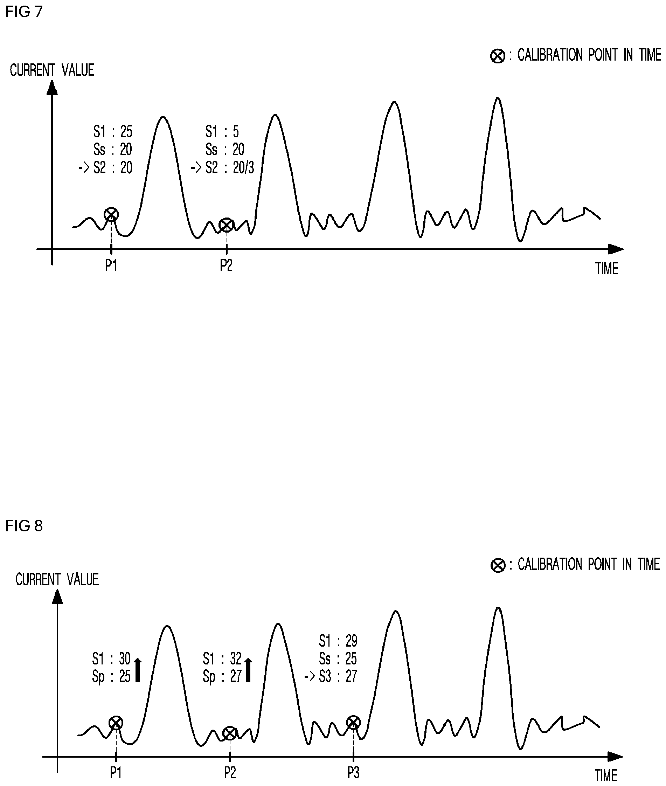

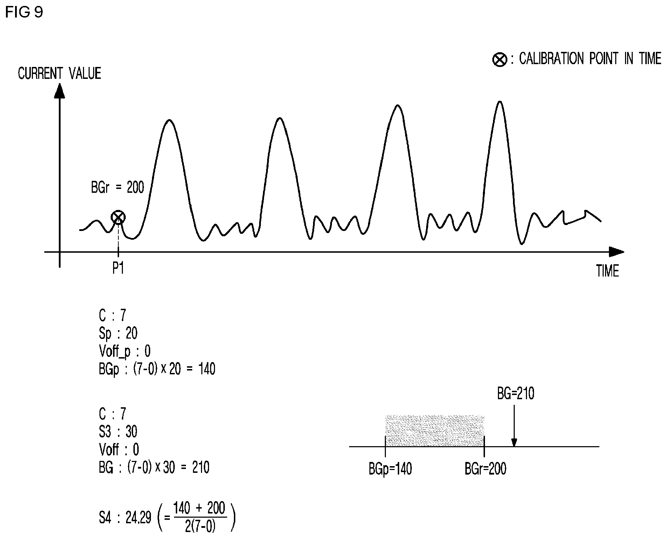

FIG. 6 is a flowchart illustrating a method for calibrating sensitivity according to one embodiment, FIG. 7 is an exemplary view of adjusting sensitivity based on coding information of a sensor according to one embodiment, FIG. 8 is an exemplary view of adjusting sensitivity based on a difference from a previous sensitivity according to one embodiment, and FIGS. 9 to 11 are exemplary views of adjusting sensitivity based on a specific range formed by a reference glucose value and a previous glucose value obtained from a previous sensitivity according to one embodiment.

Referring to FIG. 6, a method for calibrating sensitivity in a terminal constituting a CGMS according to one embodiment is shown. Although sensitivity calibration is described below as being performed in a terminal, the present invention is not limited thereto, and depending on the configuration, sensitivity calibration may also be performed in a host- for example, a server- connected to a sensor transmitter or a terminal through a wired or wireless network. A controller of the terminal may first determine sensitivity necessary for generating a glucose value from a current value and may finally apply the current value to the determined sensitivity to generate a glucose value.

To this end, the controller of the terminal may first obtain an offset value for determining sensitivity (step S601). The offset value may be a value that compensates for a change in sensitivity due to a decrease in a current value.

Then, the controller may determine sensitivity from a current value, in which the offset value is reflected, and a reference glucose value according to Mathematical Equation 2 (step S603). The sensitivity determined in this case may not be the final sensitivity and may be a sensitivity in which only the offset value is simply reflected before undergoing an adjusting step. Hereinafter, for convenience of description, such sensitivity will be referred to as "first sensitivity." Also, the current value is a type of value measured by a sensor included in a sensor module and may also be referred to as " sensor data (or analyte value)".

First, adjustment of the first sensitivity may be performed. The controller of the terminal may determine a second sensitivity from the first sensitivity and a predetermined sensitivity depending on the sensor (step S605). The predetermined sensitivity depending on the sensor may be a predetermined sensitivity for a single sensor or a predetermined sensitivity for a group- lot- which is a collection of a plurality of sensors. The sensitivity for a group of sensors may be understood as a specific sensitivity that represents the entire group instead of one sensor. Hereinafter, for convenience of description, the predetermined sensitivity depending on the sensor may be referred to as "sensor sensitivity."

Here, the sensor sensitivity may include ideal sensitivity that is expected from a sensor or a group of sensors under ideal conditions. For example, the sensor sensitivity may be understood as sensitivity that a sensor or a group of sensors should have under ideal conditions such as a laboratory environment. Alternatively, the sensor sensitivity may be understood as predetermined sensitivity in a factory when a sensor or group of sensors is manufactured. When the terminal is used, coding is necessary for initialization, and the sensor sensitivity may be included in coding information in a coding process and input and/or stored in the terminal.

Referring to FIG. 7, an example in which the second sensitivity is determined from the first sensitivity and the sensor sensitivity is shown. The controller of the terminal may reflect the sensor sensitivity to adjust the first sensitivity, and as a result thereof, may determine the second sensitivity. However, the controller of the terminal may reflect the sensor sensitivity in different ways according to some cases.

First, the controller may compare the first sensitivity and the sensor sensitivity. When the first sensitivity is higher than the sensor sensitivity, the controller may determine the sensor sensitivity as the second sensitivity. For example, in FIG. 7, when the first sensitivity S1 is 25 and the sensor sensitivity Ss, which is a sensitivity based on coding information, is 20 at a first calibration point in time P1, the controller may determine the second sensitivity S2 as 20. Hereinafter, for convenience of description, units will be omitted. The controller may trust the sensor sensitivity Ss more and determine the sensor sensitivity Ss as the second sensitivity S2.

Otherwise, that is, when the first sensitivity is lower than the sensor sensitivity, the controller may process the sensor sensitivity and determine the processed sensor sensitivity as the second sensitivity. For example, in FIG. 7, when the first sensitivity S1 is 5 and the sensor sensitivity Ss, which is a sensitivity based on coding information of the sensor, is 20 at a second calibration point in time P2, the controller may process the sensor sensitivity Ss to obtain a value that corresponds to 1/3 of the sensor sensitivity Ss and may determine 20/3($\approx$6.7), which is the value corresponding to 1/3 of the sensor sensitivity Ss, as the second sensitivity S2. The controller may trust the sensor sensitivity Ss more and determine a value obtained by processing the sensor sensitivity Ss as the second sensitivity S2.

When the first sensitivity and the sensor sensitivity are the same, the controller may determine the first sensitivity as is as the second sensitivity.

Referring back to FIG. 6, adjustment of the second sensitivity may be performed. The controller of the terminal may determine a third sensitivity by adjusting the second sensitivity based on reliability of the first sensitivity that is based on a difference between the first sensitivity and a previously determined sensitivity (step S607). Here, the previously determined sensitivity may refer to the final sensitivity at a previous point in time, and the previous point in time may refer to a specific point in time in the past that is before the current point in time at which the sensitivity is about to be calibrated. The controller of the terminal may have obtained a glucose value based on the previously determined sensitivity even at the previous point in time. Hereinafter, for convenience of description, the previously determined sensitivity may be referred to as "previous sensitivity."

Specifically, when the first sensitivity is higher or lower than the previous sensitivity continuously- at least two consecutive times or more, the controller of the terminal may trust the first sensitivity and determine that the reliability of the first sensitivity is high. Then, the controller may obtain a combined sensitivity in which the first sensitivity and the second sensitivity are reflected and may determine the combined sensitivity as the third sensitivity. The controller may average the first sensitivity and the second sensitivity to obtain the combined sensitivity.

Referring to FIG. 8, an example in which the second sensitivity is adjusted to determine the third sensitivity is shown. For example, in FIG. 8, when the first sensitivity S1 is 30 and the previous sensitivity Sp is 25 at a first calibration point in time P1, and the first sensitivity S1 is 32 and the previous sensitivity Sp is 27 at a second calibration point in time P2, since the first sensitivity S1 has a higher value than the previous sensitivity Sp at two consecutive calibration points in time, the controller may determine that reliability of the first sensitivity S1 having a value of 29 at a third calibration point in time P3 is high. Then, at the third calibration point in time P3, the controller may obtain the combined sensitivity from 29, which is the first sensitivity S1, and 25, which is the second sensitivity S2. The controller may obtain 27(=(29+25)/2), which is an average value of 29, which is the first sensitivity S1, and 25, which is the second sensitivity S2, as the combined sensitivity. The controller may determine 27, which is the combined sensitivity, as the third sensitivity S3.

When the first sensitivity is continuously higher or lower than the previous sensitivity as described above, the first sensitivity may be considered reasonable, and unlike in step S605, the first sensitivity may be reflected more than the second sensitivity.

Referring back to FIG. 6, adjustment of the third sensitivity may be performed. The controller of the terminal may obtain a glucose value from the offset value and the third sensitivity and may determine a fourth sensitivity from the third sensitivity based on whether the obtained glucose value is within a specific range (step S609). When the obtained glucose value is(or falls) within the specific range, the controller may consider the third sensitivity reasonable and may determine the third sensitivity as is as the fourth sensitivity. Otherwise, the controller may consider the third sensitivity unreasonable, obtain an adjusted sensitivity instead of the third sensitivity, and determine the adjusted sensitivity as the fourth sensitivity.

Here, the specific range may be specified by a previous glucose value and a reference glucose value. The controller may obtain the previous glucose value from a current value of the current point in time, the previous sensitivity, and a previously obtained offset value- a previous offset value. Also, the controller may obtain the reference glucose value- the reference glucose value used in determining the first sensitivity in step S601- from the outside. The controller may determine whether a glucose value obtained from the current value of the current point in time, an offset value of the current point in time, and the third sensitivity is within the range defined by the previous glucose value and the reference glucose value.

Referring to FIG. 9, one example in which the fourth sensitivity is determined from the third sensitivity based on whether the glucose value obtained from the third sensitivity is within a specific range is shown. In the present example, it may be assumed that the controller determines the fourth sensitivity at the first calibration point in time P1. First, the controller may specify the specific range and determine the validity of the third sensitivity.

When a current value C is 7, a previous sensitivity Sp is 20, and a previous offset value Voff_p is 0 at the first calibration point in time P1, a previous glucose value BGp may be $140(=(7-0)\times20)$. Here, the previous sensitivity Sp and the previous offset value Voff_p may be a sensitivity and an offset value that are determined before sensitivity calibration is performed at the first calibration point in time P1. When a reference glucose value BGr is 200 at the first calibration point in time P1, a range of a glucose value based on the third sensitivity for the glucose value to be valid, that is, a specific range specified by the previous glucose value and the reference glucose value, may correspond to 140 to 200.

Also, since the current value C at the first calibration point in time P1 is 7, an offset value Voff at the first calibration point in time P1 is 0, and a third sensitivity S3 is 30, from these, the controller may obtain $210(=(7-0)\times30)$ as a glucose value BG. Since the glucose value BG is 210 and is out of (or deviates from) the specific range of 140 to 200, the controller may consider the third sensitivity S3 invalid and may obtain an adjusted sensitivity to determine a fourth sensitivity.

The controller may obtain the adjusted sensitivity through Mathematical Equation 3 below using a current value (at the current point in time), an offset value (at the current point in time), a previous glucose value, and a reference glucose value.

[Mathematical Equation 3]

$$Sa = \frac{BGp + BGr}{2} \frac{1}{C - Voff}$$

According to the present example, since the current value C at the first calibration point in time P1 is 7, the offset value Voff at the first calibration point in time P1 is 0, the previous glucose value BGp is 140, and the reference glucose value BGr is 200, the adjusted sensitivity Sa may be 24.29. The controller may determine the adjusted sensitivity Sa as the fourth sensitivity. The controller may determine 24.29 as the fourth sensitivity.

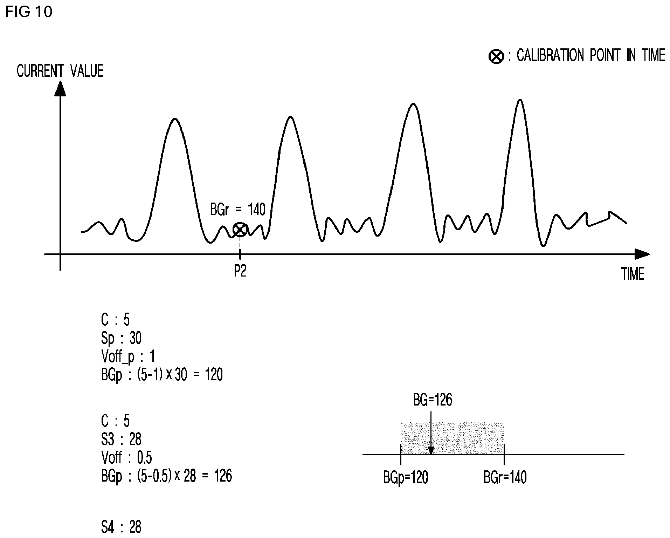

Referring to FIG. 10, another example in which the fourth sensitivity is determined from the third sensitivity based on whether the glucose value obtained from the third sensitivity is within a specific range is shown. In the present example, it may be assumed that the controller determines the fourth sensitivity at the second calibration point in time P2. First, the controller may specify the specific range and determine the validity of the third sensitivity.

When a current value C is 5, a previous sensitivity Sp is 30, and a previous offset value Voff_p is 1 at the second calibration point in time P2, a previous glucose value BGp may be 120(=(5-1)×30). Here, the previous sensitivity Sp and the previous offset value Voff_p may be a sensitivity and an offset value that are determined before sensitivity calibration is performed at the second calibration point in time P2. When a reference glucose value BGr is 140 at the second calibration point in time P2, a range of a glucose value based on the third sensitivity for the glucose value to be valid, that is, a specific range specified by the previous glucose value and the reference glucose value, may correspond to 120 to 140.

Also, since the current value C at the second calibration point in time P2 is 5, an offset value Voff at the second calibration point in time P2 is 0.5, and the third sensitivity S3 is 28, from these, the controller may obtain 126(=(5-0.5)×28) as a glucose value BG. Since the glucose value BG is 126 and is within the specific range of 120 to 140, the controller may consider the third sensitivity S3 valid and determine the third sensitivity S3 as the fourth sensitivity. Therefore, in the present example, the fourth sensitivity may be 28.

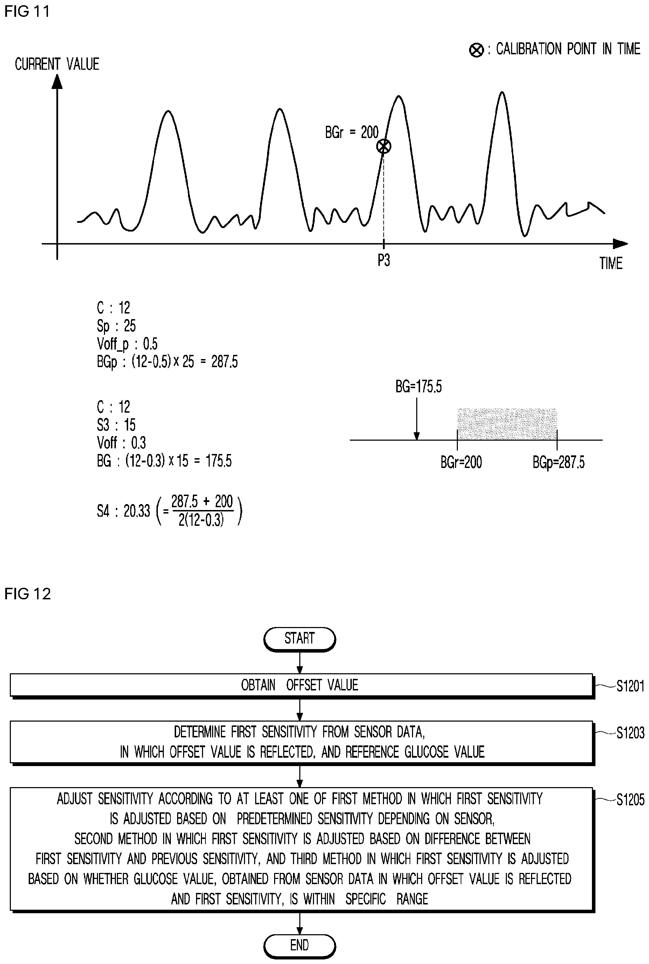

Referring to FIG. 11, still another example in which the fourth sensitivity is determined from the third sensitivity based on whether the glucose value obtained from the third sensitivity is within a specific range is shown. In the present example, it may be assumed that the controller determines the fourth sensitivity at the third calibration point in time P3. First, the controller may specify the specific range and determine the validity of the third sensitivity.

When a current value C is 12, a previous sensitivity Sp is 25, and a previous offset value Voff_p is 0.5 at the third calibration point in time P3, a previous glucose value BGp may be 287.5(=(12-0.5)×25). Here, the previous sensitivity Sp and the previous offset value Voff_p may be a sensitivity and an offset value that are determined before sensitivity calibration is performed at the third calibration point in time P3. When a reference glucose value BGr is 200 at the third calibration point in time P3, a range of a glucose value based on the third sensitivity for the glucose value to be valid, that is, a specific range specified by the previous glucose value and the reference glucose value, may correspond to 200 to 287.5.

Also, since the current value C at the third calibration point in time P3 is 12, an offset value Voff at the third calibration point in time P3 is 0.3, and the third sensitivity S3 is 15, from these, the controller may obtain 175.5(=(12-0.3)×15) as a glucose value BG. Since the glucose value BG is 175.5 and is out of (or deviates from) the specific range of 200 to 287.5, the controller may consider the third sensitivity S3 invalid and may obtain an adjusted sensitivity to determine a fourth sensitivity.

The controller may obtain the adjusted sensitivity through Mathematical Equation 3 above using a current value (at the current point in time), an offset value (at the current point in time), a previous glucose value, and a reference glucose value. According to the present example, since the current value C at the third calibration point in time P3 is 12, the offset value Voff at the third calibration point in time P3 is 0.3, the previous glucose value BGp is 287.5, and the reference glucose value BGr is 200, the adjusted sensitivity Sa may be 20.33. The controller may determine 20.33 as the fourth sensitivity.

FIG. 12 is a flowchart illustrating a method for calibrating sensitivity according to another embodiment.

Referring to FIG. 12, a method for calibrating sensitivity in a terminal constituting a CGMS according to another embodiment is shown. The controller of the terminal may first obtain an offset value for determining sensitivity (step S1201).

Also, the controller may determine a first sensitivity from a sensor data (or current value), in which the obtained offset value is reflected, and a reference glucose value (step S1203).

Then, the controller may adjust sensitivity according to at least one of a first method in which the first sensitivity is adjusted based on sensor sensitivity-predetermined sensitivity depending on a sensor, a second method in which the first sensitivity is adjusted based on a difference between the first sensitivity and a previous sensitivity, and a third method in which the first sensitivity is adjusted based on whether a glucose value obtained from the first sensitivity is within a specific range specified by a previous glucose value and the reference glucose value (step S1205). Also, the controller may sequentially perform the first to third methods one by one to adjust sensitivity or may selectively perform only one method to adjust sensitivity.

Here, the first to third methods may be the same as one embodiment and may have a few differences therefrom. The controller may use the first method alone to calibrate sensitivity. Then, in the same manner as in one embodiment, the controller may adjust the first sensitivity based on the sensor sensitivity to determine the final sensitivity.

Also, the controller may use the second method alone to calibrate sensitivity. When the second method is not used right after the first method, a sensitivity adjusted based on the sensor sensitivity may not be present in the second method. Then, when the first sensitivity is continuously higher or lower than the previous sensitivity, the controller may determine the first sensitivity as is as the final sensitivity.

Also, the controller may use the third method alone to calibrate sensitivity. Then, in the same manner as in one embodiment, the controller may determine the first sensitivity as the final sensitivity based on whether a glucose value obtained from the first sensitivity is within a specific range specified by a previous glucose value and a reference glucose value or may obtain an adjusted sensitivity according to Mathematical Equation 3 and determine the adjusted sensitivity as the final sensitivity.

The controller may end the sensitivity calibration process once the controller determines the first sensitivity adjusted according to at least one or more of the first to third methods as the final sensitivity. The controller may generate a glucose value based on the final sensitivity, an offset value, and a sensor data (current value).

As described above, according to the present embodiments, accurate sensitivity can be obtained by calibrating sensitivity.

Also, according to the present embodiments, the accuracy of sensitivity can be improved by adjusting sensitivity through several steps.

Aspects of subjects described herein may be described in the context of computer-executable instructions, such as program modules, being executed by computers. Generally, program modules include routines, programs, objects, components, data structures, and the like, which perform particular tasks or implement particular abstract data types.

Alternatively, or in addition, the functions described herein can be performed, at least in part, by one or more hardware logic components. By way of example and not limitation, illustrative types of hardware logic components that can be used include field-programmable gate arrays (FPGAs), application-specific integrated circuits (ASICs), application-specific standard products (ASSPs), system-on-a-chip systems (SOCs), complex programmable logic devices (CPLDs), and the like.

Meanwhile, the embodiments disclosed herein may be implemented in the form of recording media that store computer-executable programs and/or instructions. The instructions may be stored in the form of program code, and when executed by a processor, may generate a program module to perform operations of the disclosed embodiments. The recording media may be implemented as computer-readable recording media.

The computer-readable recording media include any type of recording media in which instructions that can be deciphered by computers are stored. Examples of the computer-readable recording media may include a read only memory (ROM), a random access memory (RAM), a magnetic tape, a magnetic disk, a flash memory, an optical data storage device, and the like.

Embodiments of the present invention have been described above, but those of ordinary skill in the art may make various modifications and changes to the present invention by adding other components, changing components, or omitting components within the scope not departing from the spirit of the present invention described in the claims, and such modifications and changes also belong to the scope of rights of the present invention.

## Claims

1. A method for calibrating sensitivity for glucose measurement, the method comprising:

   obtaining an offset value for determining sensitivity;
   determining a first sensitivity from a sensor data and a reference glucose value, based on the offset value;
   determining a second sensitivity from the first sensitivity and a predetermined sensitivity depending on a sensor;
   determining a third sensitivity by adjusting the second sensitivity based on reliability of the first sensitivity that is based on a difference between the first sensitivity and a previous sensitivity;
   determining a fourth sensitivity from the third sensitivity based on whether a glucose value obtained from the offset value and the third sensitivity is within a specific range; and
   determining the fourth sensitivity as the sensitivity.

2. The method of claim 1, wherein the determining of the second sensitivity includes, when the first sensitivity is higher than the predetermined sensitivity depending on the sensor, determining the predetermined sensitivity depending on the sensor as the second sensitivity.

3. The method of claim 1, wherein the determining of the second sensitivity includes, when the first sensitivity is lower than the predetermined sensitivity depending on the sensor, processing the predetermined sensitivity depending on the sensor; and determining a processed sensitivity as the second sensitivity.

4. The method of claim 1, wherein the predetermined sensitivity depending on the sensor includes a predetermined ideal sensitivity for the sensor or a group of sensors.

5. The method of claim 1, wherein the determining of the third sensitivity by adjusting the second sensitivity based on the reliability of the first sensitivity includes:

   when the difference occurs continuously, determining that the reliability of the first sensitivity is high;
   obtaining a combined sensitivity from the first sensitivity and the second sensitivity; and
   determining the combined sensitivity as the third sensitivity.

6. The method of claim 5, wherein the obtaining of the combined sensitivity includes averaging the first sensitivity and the second sensitivity.

7. The method of claim 1, wherein the specific range is specified by a previous glucose value, which is obtained from a obtained offset value and the previous sensitivity, and the reference glucose value.

8. The method of claim 7, wherein the determining of the fourth sensitivity from the third sensitivity includes, when the obtained glucose value is within the specific range, determining the third sensitivity as the fourth sensitivity.

9. The method of claim 7, wherein the determining of the fourth sensitivity from the third sensitivity includes:

   when the obtained glucose value is out of the specific range, obtaining an adjusted sensitivity from a sensor data, in which the offset value is reflected, the previous glucose value, and the reference glucose value; and
   determining the adjusted sensitivity as the fourth sensitivity.

10. The method of claim 9, wherein the obtaining of the adjusted sensitivity includes obtaining the adjusted sensitivity using an average value of the previous glucose value and the reference glucose value.

11. A method for calibrating sensitivity for glucose measurement, the method comprising:

   obtaining an offset value;
   determining a sensitivity from a sensor data, in which the offset value is reflected, and a reference glucose value; and
   adjusting the sensitivity according to at least one of a first method in which the sensitivity is adjusted based on a predetermined sensitivity depending on a sensor, a second method in which the sensitivity is adjusted based on a difference between the sensitivity and a previous sensitivity, and a third method in which the sensitivity is adjusted based on whether a glucose value obtained from the sensor data in which the offset value is reflected and the sensitivity is within a specific range.

12. The method of claim 11, wherein the adjusting of the sensitivity according to at least one of the first to third methods includes adjusting the sensitivity by sequentially applying the first to third methods.

FIG 1

FIG 2

300

FIG 3

FIG 4

100

110

SENSOR MODULE

SENSOR COMMUNICATION UNIT

120

SENSOR CONTROLLER

130

SENSOR STORAGE

140

FIG 5

200

210

UTPUT UNIT

COMMUNICATION UNIT

220

CONTROLLER

230

STORAGE

240

FIG 6

```
                        ┌─────────────┐
                        │   START     │
                        └──────┬──────┘
                               │
                               ▼
┌──────────────────────────────────────────────────────────────┐
│        OBTAIN OFFSET VALUE FOR DETERMINING SENSITIVITY         │──S601
└──────────────────────────────────────────────────────────────┘
                               │
                               ▼
┌──────────────────────────────────────────────────────────────┐
│           DETERMINE FIRST SENSITIVITY FROM SENSOR DATA,        │──S603
│   IN WHICH OFFSET VALUE IS REFLECTED, AND REFERENCE GLUCOSE VALUE │
└──────────────────────────────────────────────────────────────┘
                               │
                               ▼
┌──────────────────────────────────────────────────────────────┐
│          DETERMINE SECOND SENSITIVITY FROM FIRST SENSITIVITY    │──S605
│          AND PREDETERMINED SENSITIVITY DEPENDING ON SENSOR      │
└──────────────────────────────────────────────────────────────┘
                               │
                               ▼
┌──────────────────────────────────────────────────────────────┐
│  DETERMINE THIRD SENSITIVITY BY ADJUSTING SECOND SENSITIVITY BASED │──S607
│   ON RELIABILITY BASED ON DIFFERENCE BETWEEN FIRST SENSITIVITY  │
│                    AND PREVIOUS  SENSITIVITY                    │
└──────────────────────────────────────────────────────────────┘
                               │
                               ▼
┌──────────────────────────────────────────────────────────────┐
│    DETERMINE FOURTH SENSITIVITY FROM THIRD SENSITIVITY BASED    │──S609
│   ON WHETHER GLUCOSE VALUE  OBTAINED FROM OFFSET VALUE          │
│          AND THIRD SENSITIVITY IS WITHIN SPECIFIC RANGE         │
└──────────────────────────────────────────────────────────────┘
                               │
                               ▼
┌──────────────────────────────────────────────────────────────┐
│       FINALLY DETERMINE FOURTH SENSITIVITY AS SENSITIVITY      │──S611
└──────────────────────────────────────────────────────────────┘
                               │
                               ▼
                        ┌─────────────┐
                        │    END      │
                        └─────────────┘
```

FIG 7

⊗ : CALIBRATION POINT IN TIME

CURRENT VALUE

S1 : 25
Ss : 20
-> S2 : 20

S1 : 5
Ss : 20
-> S2 : 20/3

P1

P2

TIME

FIG 8

⊗ : CALIBRATION POINT IN TIME

CURRENT VALUE

S1 : 30
Sp : 25

S1 : 32
Sp : 27

S1 : 29
Ss : 25
-> S3 : 27

P1

P2

P3

TIME

16

FIG 9

⊗ : CALIBRATION POINT IN TIME

CURRENT VALUE

BGr = 200

P1

TIME

C : 7
Sp : 20
Voff_p : 0
BGp : (7-0) x 20 = 140

C : 7
S3 : 30
Voff : 0
BG : (7-0) x 30 = 210

$S4 : 24.29 \left( = \dfrac{140 + 200}{2(7-0)} \right)$

BG=210

BGp=140          BGr=200

FIG 10

⊗ : CALIBRATION POINT IN TIME

CURRENT VALUE

BGr = 140

P2

TIME

C : 5
Sp : 30
Voff_p : 1
BGp : (5-1) × 30 = 120

C : 5
S3 : 28
Voff : 0.5
BGp : (5-0.5) × 28 = 126

S4 : 28

BG=126

BGp=120     BGr=140

## FIG 11

⊗ : CALIBRATION POINT IN TIME

CURRENT VALUE

BGr = 200

P3

TIME

C : 12
Sp : 25
Voff_p : 0.5
BGp : (12-0.5) × 25 = 287.5

C : 12
S3 : 15
Voff : 0.3
BG : (12-0.3) × 15 = 175.5

$$S4 : 20.33 \left( = \frac{287.5 + 200}{2(12-0.3)} \right)$$

BG=175.5

BGr=200    BGp=287.5

## FIG 12

START

OBTAIN OFFSET VALUE — S1201

DETERMINE FIRST SENSITIVITY FROM SENSOR DATA,
IN WHICH OFFSET VALUE IS REFLECTED, AND REFERENCE GLUCOSE VALUE — S1203

ADJUST SENSITIVITY ACCORDING TO AT LEAST ONE OF FIRST METHOD IN WHICH FIRST SENSITIVITY
IS ADJUSTED BASED ON PREDETERMINED SENSITIVITY DEPENDING ON SENSOR,
SECOND METHOD IN WHICH FIRST SENSITIVITY IS ADJUSTED BASED ON DIFFERENCE BETWEEN
FIRST SENSITIVITY AND PREVIOUS SENSITIVITY, AND THIRD METHOD IN WHICH FIRST SENSITIVITY IS ADJUSTED
BASED ON WHETHER GLUCOSE VALUE, OBTAINED FROM SENSOR DATA IN WHICH OFFSET VALUE IS REFLECTED
AND FIRST SENSITIVITY, IS WITHIN SPECIFIC RANGE — S1205

END

Europäisches
Patentamt

European
Patent Office

Office européen
des brevets

# EUROPEAN SEARCH REPORT

**Application Number**

EP 24 18 1827

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/283960 A1 (BUDIMAN ERWIN SATRYA [US]) 8 November 2012 (2012-11-08) * paragraphs [0076] - [0116] * | 1-12 | INV. A61B5/145 A61B5/1495 A61B5/00 A61B5/1486 |
| X | US 2009/112478 A1 (MUELLER JR JOHN C [US] ET AL) 30 April 2009 (2009-04-30) * paragraphs [0094] - [0149] * | 1-12 | |
| X | US 2011/184268 A1 (TAUB MARC BARRY [US]) 28 July 2011 (2011-07-28) * paragraphs [0063] - [0088] * | 1-12 | |
| X | US 2016/183855 A1 (VANSLYKE STEPHEN J [US] ET AL) 30 June 2016 (2016-06-30) * paragraphs [0131] - [0192] * | 1-12 | |
| X | US 2009/036760 A1 (HAYTER GARY [US]) 5 February 2009 (2009-02-05) * paragraphs [0117] - [0124], [0146] - [0163] * | 1-12 | TECHNICAL FIELDS SEARCHED (IPC) |
| X | US 2021/186383 A1 (SIMPSON PETER C [US] ET AL) 24 June 2021 (2021-06-24) * paragraphs [0988] - [1026] * | 1-12 | A61B |

The present search report has been drawn up for all claims

| Place of search | Date of completion of the search | Examiner |
|---|---|---|
| The Hague | 15 October 2024 | Crisan, Carmen-Clara |

EPO FORM 1503 03.82 (P04C01)

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

# EP 4 477 147 A1

## ANNEX TO THE EUROPEAN SEARCH REPORT
## ON EUROPEAN PATENT APPLICATION NO.

EP 24 18 1827

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2024

| Patent document cited in search report | | | Publication date | Patent family member(s) | | | Publication date |
|---|---|---|---|---|---|---|---|
| US 2012283960 | A1 | | 08-11-2012 | EP | 2381838 | A1 | 02-11-2011 |
| | | | | US | 2010191085 | A1 | 29-07-2010 |
| | | | | US | 2012283960 | A1 | 08-11-2012 |
| | | | | US | 2014005968 | A1 | 02-01-2014 |
| | | | | US | 2019035488 | A1 | 31-01-2019 |
| | | | | US | 2023255518 | A1 | 17-08-2023 |
| | | | | WO | 2010088569 | A1 | 05-08-2010 |
| US 2009112478 | A1 | | 30-04-2009 | CA | 2702428 | A1 | 07-05-2009 |
| | | | | CA | 2782491 | A1 | 07-05-2009 |
| | | | | DK | 2207473 | T3 | 15-06-2015 |
| | | | | EP | 2207473 | A2 | 21-07-2010 |
| | | | | EP | 2896357 | A1 | 22-07-2015 |
| | | | | US | 2009112478 | A1 | 30-04-2009 |
| | | | | US | 2010268477 | A1 | 21-10-2010 |
| | | | | WO | 2009058792 | A2 | 07-05-2009 |
| US 2011184268 | A1 | | 28-07-2011 | EP | 2558970 | A1 | 20-02-2013 |
| | | | | US | 2011184268 | A1 | 28-07-2011 |
| | | | | WO | 2012170000 | A1 | 13-12-2012 |
| US 2016183855 | A1 | | 30-06-2016 | AU | 2014242360 | A1 | 04-06-2015 |
| | | | | AU | 2017203366 | A1 | 08-06-2017 |
| | | | | AU | 2019283801 | A1 | 16-01-2020 |
| | | | | AU | 2022201202 | A1 | 17-03-2022 |
| | | | | AU | 2024204270 | A1 | 11-07-2024 |
| | | | | CA | 2892266 | A1 | 02-10-2014 |
| | | | | CN | 105408902 | A | 16-03-2016 |
| | | | | CN | 109431472 | A | 08-03-2019 |
| | | | | EP | 2973081 | A2 | 20-01-2016 |
| | | | | EP | 3806103 | A1 | 14-04-2021 |
| | | | | US | 2014278189 | A1 | 18-09-2014 |
| | | | | US | 2016183855 | A1 | 30-06-2016 |
| | | | | US | 2019261905 | A1 | 29-08-2019 |
| | | | | US | 2020337607 | A1 | 29-10-2020 |
| | | | | WO | 2014158327 | A2 | 02-10-2014 |
| US 2009036760 | A1 | | 05-02-2009 | US | 2009036760 | A1 | 05-02-2009 |
| | | | | US | 2017181679 | A1 | 29-06-2017 |
| | | | | US | 2020121231 | A1 | 23-04-2020 |
| | | | | US | 2022273203 | A1 | 01-09-2022 |
| | | | | US | 2023293057 | A1 | 21-09-2023 |
| US 2021186383 | A1 | | 24-06-2021 | US | 11000215 | B1 | 11-05-2021 |
| | | | | US | 11020031 | B1 | 01-06-2021 |
| | | | | US | 2019357827 | A1 | 28-11-2019 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 24 18 1827

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2024

| Patent document cited in search report | Publication date | Patent family member(s) | | Publication date |
|---|---|---|---|---|
| | | US | 2021259587 A1 | 26-08-2021 |
| | | US | 2021282682 A1 | 16-09-2021 |
| | | US | 2022167891 A1 | 02-06-2022 |

EPO FORM P0459

For more details about this annex : see Official Journal of the European Patent Office, No. 12/82

page 2 of 2

**EP 4 477 147 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230075334